(19) Europäisches Patentamt
European Patent Office
Office européen des brevets

(11) **EP 4 502 177 A1**

(12) **EUROPEAN PATENT APPLICATION**
published in accordance with Art. 153(4) EPC

(43) Date of publication:
**05.02.2025 Bulletin 2025/06**

(21) Application number: **23774011.3**

(22) Date of filing: **24.03.2023**

(51) International Patent Classification (IPC):
**C12Q 1/6806** (2018.01)   **C12Q 1/6851** (2018.01)
**C12Q 1/6855** (2018.01)   **C40B 70/00** (2006.01)
**C12Q 1/6869** (2018.01)

(52) Cooperative Patent Classification (CPC):
**C12Q 1/6806; C12Q 1/6851; C12Q 1/6855;
C40B 70/00;** C12Q 1/6869

(86) International application number:
**PCT/CN2023/083705**

(87) International publication number:
**WO 2023/179766 (28.09.2023 Gazette 2023/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.03.2022 CN 202210303736**

(71) Applicant: **Nanjing Legend Biotech Co., Ltd.
Nanjing, Jiangsu 211100 (CN)**

(72) Inventors:
• **LIU, Jin
  Nanjing, Jiangsu 211100 (CN)**
• **CAO, Qiansheng
  Nanjing, Jiangsu 211100 (CN)**
• **CHEN, Qingqing
  Nanjing, Jiangsu 211100 (CN)**
• **ZHANG, Min
  Nanjing, Jiangsu 211100 (CN)**

(74) Representative: **Murgitroyd & Company
Murgitroyd House
165-169 Scotland Street
Glasgow G5 8PL (GB)**

Remarks:
The complete document including Reference
Table(s) and the Sequence Listing(s) can be
downloaded from the EPO website

(54) **METHOD FOR PREPARING DNA LIBRARY AND DETECTING RETROVIRAL INTEGRATION SITE**

(57) The present disclosure belongs to the field of molecular biology, particularly to the technical field of gene analysis and detection, and specifically relates to a method for preparing a DNA library and a method for detecting a retroviral integration site. Specifically, the method for preparing the DNA library comprises the following steps: 1) fragmenting a genomic DNA from a retrovirus-infected cell to obtain DNA fragments; 2) subjecting the DNA fragments to end-repair, A-tailing, and ligation with an adapter to obtain a ligation product, wherein the adapter is an asymmetric double-strand adapter comprising a long-strand sequence and a short-strand sequence, wherein the long-strand sequence sequentially comprises, from a 5' end to a 3' end, a fixed sequence, a random UMI sequence, and an amplification primer binding sequence, and the short-strand sequence comprises a sequence complementary to the fixed sequence; and other steps. The detection method of the present disclosure has extremely high sensitivity, thus having good application potential.

EP 4 502 177 A1

3'LTR or ApoB target sequence

Fragment to ~1000 bp with M220 sonication

3'LTR or ApoB target sequence

Perform end-repair and A-tailing, and add an asymmetric adapter

Sample index

UMI

P5 adapter   3'LTR or ApoB target sequence

P5 adapter

1st amplification

LTR or ApoB_specific_F_1

P5_adapter_primer_1

2nd amplification (nested PCR)

LTR or ApoB_specific_F_2

P5_adapter_primer_2

3rd amplification (add P5/P7 adapters)

LG_Dual_Index_Adapter_i7

LG_Dual_Index_Adapter_i5

*FIG. 1*

**Description**

Cross-Reference to Related Applications

The present application claims the benefit of priority for Chinese Patent Application No. 202210303736.0 filed on March 24, 2022, the content of which is incorporated herein by reference in their entirety.

Technical Field

The present disclosure belongs to the field of molecular biology, particularly to the technical field of gene analysis and detection, and specifically relates to a method for preparing a DNA library and a method for detecting a retroviral integration site. In particular, the present disclosure relates to a method for absolute quantitative detection of a retroviral (e.g., lentiviral) integration site.

Background Art

Gene and cell therapy is an effective means of treating genetic diseases and other malignant diseases, for example, CD19 and BCMA CAR-T have been approved for clinical use in the treatment of hematological tumors. Currently, CAR-T products marketed worldwide use viral vector transduction, such as Kymriah from Novartis, which is developed and produced on the basis of a lentiviral vector, and Yescarta from Kite/Gilead, which uses a γ-retroviral vector transduction system.

According to the current international virus taxonomy criteria, the *Retroviridae* family is divided into 7 genera: *Alpharetrovirus*, *Betaretrovirus*, *Gammaretrovirus*, *Deltaretrovirus*, *Epsilonretrovirus*, *Lentivirus* and *Spumavirus*, wherein the *Lentivirus* is subdivided into 5 groups, including bovine lentivirus group, equine lentivirus group, feline lentivirus group, ovine lentivirus group, and human lentivirus group. HIV is defined in the "large family" of viruses as belonging to the human immunodeficiency virus group in the genus *Lentivirus* of the *Retroviridae* family.

Lentiviruses, a subset of retroviruses, are one of the most common and useful types of viruses in research. Lentiviruses can transduce dividing and non-dividing cells without producing an obvious immune response. These viruses can also be stably integrated into a host genome, thereby enabling long-term transgene expression. There are some safety considerations that need to be assessed when using lentiviruses: these viruses are HIV-1-based and may require additional laboratory biosafety procedures. In addition, these viruses may inactivate tumor suppressor genes or activate proto-oncogene expression due to their random integration into the host genome, leading to the risk of carcinogenesis (Hacein-Bey-Abina S et al., science, 2003, 302 (5644): 415-419.; Six E et al., Molecular Therapy. 50 HAMPSHIRE ST, FLOOR 5, CAMBRIDGE, MA 02139 USA: CELL PRESS, 2017, 25 (5): 347-348; Bluebird bio reports second quarter financial results and provides operational update. News release. bluebirdbio. August 9, 2021).

γ-retrovirus is an RNA virus consisting of its genome and several structural proteins and enzymatic proteins, including reverse transcriptases and integrases. Once entering the target cell, the virus uses two reverse transcriptases to produce a DNA provirus. This provirus is then integrated into the host genome by accompanying integrase proteins.

When scientists discuss retroviruses, they usually refer to a subset of retroviruses, called γ-retroviruses. γ-retroviruses can encapsulate relatively large amounts of DNA (up to 8 kb), and infection results in long-term transgene expression. Some disadvantages of γ-retroviruses are that they only transduce dividing cells (because they can enter the nucleus only upon nuclear envelope breakdown during mitosis). In addition, γ-retroviruses are randomly integrated into the host genome, which may lead to tumorigenesis (referred to as insertion mutation).

In preclinical studies and clinical applications, the assessment of viral integration sites and associated safety is a must. For example, both the US FDA and the European EMA require the detection of viral integration sites and integration frequency in gene and cell therapy to clarify the safety of this therapy (O'Leary M C et al., Clinical Cancer Research, 2019, 25 (4): 1142-1146; U.S. Department of Health and Human Services. Long Term Follow-Up After Administration of Human Gene Therapy Products; Guidance for Industry. Jan 30, 2020.; European medicines agency. Guideline on the quality, non-clinical and clinical aspects of gene therapy medicinal products. 22 March, 2018.). The domestic "Technical Guidelines for Non-clinical Research of Genetically Modified Cell Therapy Products (Trial)" (November 2021) also clarifies the risk assessment of integration/insertion sites as part of the preclinical safety evaluation.

In addition, the detection technology of viral integration sites can also be used to track the clonal evolution of engineered immune cells after cell therapy. Compared to the detection method of using CAR-T-specific TCR (CAR-T therapy) to track the proportion of CAR-T cells, the method for detecting viral integration sites has the following advantages: 1) no need to identify and distinguish specific TCR sequences for CAR-T and endogenous T cells prior to tracking at serial time points; 2) no need to sort CAR-T in samples; 3) no need for fresh blood/tissue samples; and 4) low cost, short experimental period, etc.

At present, there are several methods for detecting viral integration sites: 1) probe method, which is relatively low in sensitivity and difficult to implement in samples with low integration ratio; 2) linear amplification-mediated (LAM)-PCR, on which the existing analyses of viral integration sites are mostly based; 3) modified genome sequencing (MGS)-PCR, which has a relatively simpler process compared to LAM-PCR; and 4) amplicon method, a combination of LAM-PCR using non-restriction endonuclease cleavage and anchored multiplex PCR, which can be used for the detection of lentiviral integration sites.

However, the above-mentioned methods all have some drawbacks, such as low sensitivity and poor accuracy. In addition, none of the above-mentioned methods can achieve absolute quantification of each integration site, and the sensitivity of the method cannot be assessed when the overall copy number is unknown. Therefore, there is still a need to develop new methods for detecting retroviral integration sites.

Summary

The inventors, through intensive research and inventive efforts, have obtained a method for preparing a DNA library and the prepared DNA library and have further obtained a method for detecting a retroviral integration site. The inventors have surprisingly found that the detection method of the present disclosure has extremely high sensitivity, thus having good application potential. The following invention is thus provided:

One aspect of the present disclosure relates to a method for preparing a DNA library, comprising the steps of:

1) fragmenting (for example, sonicating) a genomic DNA from a retrovirus-infected cell to obtain DNA fragments;
2) subjecting the DNA fragments to end-repair, A-tailing, and ligation with an adapter to obtain a ligation product, wherein the adapter is an asymmetric double-strand adapter comprising a long-strand sequence and a short-strand sequence, wherein the long-strand sequence sequentially comprises, from a 5' end to a 3' end, a fixed sequence, a random UMI sequence, and an amplification primer binding sequence, and the short-strand sequence comprises a sequence complementary to the fixed sequence;
3) performing a first round of PCR on the ligation product with primers for an LTR sequence, primers for an internal reference gene sequence, and primers for an adapter sequence to obtain a first round of PCR products;
4) performing a second round of PCR on the first round of PCR products with primers for the LTR sequence, primers for the internal reference gene sequence, and primers for the adapter sequence which are different from those in step 3) to obtain a second round of PCR products; and
5) performing a third round of PCR on the second round of PCR products with primers having sequencing adapters to obtain a third round of PCR products as a DNA library.

Without being bound by theory, the reaction system in which an internal reference gene is incorporated will enable the formation of a sufficient amount of DNA even when the content/frequency of integration sites in samples is particularly low (for example, 1% or less) to complete the detection with a stable reaction program. The inventors have found that, when PCR is used to amplify and enrich integration sites, in the case where the content/frequency of integration sites in samples is particularly low, if no internal reference gene is introduced, a sufficient amount of DNA for purification and use as a template for the next PCR cannot be obtained under a fixed number of PCR cycles, which will make the detection step impossible. In this case, there are usually two solutions: 1. to obtain a sufficient amount of DNA, amplification is performed at high cycle numbers, but this results in an increase in non-specific amplification, affecting the effective data and quality of the final library; 2. other methods are used to predict the total frequency of integration sites and flexibly adjust the number of cycles to complete library construction, but at this time, the library construction procedure using each batch of data is inconsistent, resulting in inconsistent library quality and impossible establishment of stable background filtering thresholds for downstream data analysis.

Without being bound by theory, the sequencing read of the internal reference gene can be used as a standard for downsample (downsample, random extraction of narrowed data) for subsequent bioinformatics analysis to ensure that the depth of sequencing for each integration site remains consistent in each experiment. When downsample of data is performed according to a certain amount of the internal reference gene, the depth of sequencing can remain as consistent as possible regardless of the number of copies of integration sites, so that a stale cutoff value (for example, 3 reads or more detected is determined to be a true result) can be used to filter background noise in each experiment. In the absence of an internal reference gene, since the frequency of integration sites in each sample is inconsistent, the actual depth of sequencing for each integration site is inconsistent in different samples when downsample is performed according to a certain amount of sequencing data, which will cause the magnitude of the background noise to be different in different samples, and therefore it is impossible to form a stable cutoff value to remove background noise.

In addition, the internal reference gene of the present disclosure can be used as a calibration value to calibrate the concentration of copy number (copy number/$\mu$g gDNA) in a sample for each integration site in the sample of the present disclosure, thereby enabling absolute quantification of each integration site.

In some embodiments of the present disclosure, in the preparation method, the retrovirus-infected cell is an engineered immune cell, and the engineered immune cell comprises a chimeric antigen receptor; preferably, the engineered immune cell is selected from a cytotoxic T cell, a helper T cell, a natural killer T cell, a γδ T cell, an NK cell, a macrophage, a B cell, an antigen presenting cell, a dendritic cell and a stem cell-induced immune cell.

In some embodiments of the present disclosure, in the preparation method, in step 1), the DNA fragment is 500-1500 bp, 800-1200 bp, 900-1100 bp or 1000 bp in length.

In some embodiments of the present disclosure, in the preparation method, in step 2), the fixed sequence is 14-30 bases in length; and/or

the random UMI sequence is 5-12 bases in length, wherein each base is independently A, G, C, or T; preferably, the random UMI sequence is 5-9 bases in length.

The random UMI sequence is formally (N)x, in which N independently represents base A, G, C or T, x is a natural number of 5-12 or 5-9, such as 5, 6, 7, 8, 9, 10, 11 or 12.

In some embodiments of the present disclosure, in the preparation method, in step 2), the 5' end of the short-strand sequence is provided with a modification that prevents primer extension, preferably an inverted dT modification or a dideoxycytidine modification.

In some embodiments of the present disclosure, in the preparation method, in step 4),

the primers for the LTR sequence in the second round of PCR are located downstream of the primers for the LTR sequence in the first round of PCR,
the primers for the internal reference gene sequence in the second round of PCR are located downstream of the primers for the internal reference gene sequence in the first round of PCR, and
the primers for the adapter sequence in the second round of PCR cover the primers for the adapter sequence in the first round of PCR and extend downstream.

In some embodiments of the present disclosure, in the preparation method, the total number of cycles in the first round of PCR, the second round of PCR and the third round of PCR is 36-63, preferably 36-45 or 38-42.

In some embodiments of the present disclosure, in the preparation method, the ratio of the number of cycles in the first round of PCR to that in the second round of PCR is (1-3) : 2, for example, 1 : 2, 3 : 2, 1 : 1 or 3 : 4;

preferably, the number of cycles in the first round of PCR and the second round of PCR is 30 and 20, 15 and 20, 10 and 20, or 15 and 15, respectively.

In some embodiments of the present disclosure, in the preparation method, the number of cycles in the third round of PCR is 6-13, for example, 6, 10 or 13;

preferably, the number of cycles in the first round of PCR, the second round of PCR and the third round of PCR is 15, 20 and 6, respectively, 10, 20 and 6, respectively, or 15, 15 and 6, respectively.

In some embodiments of the present disclosure, in the preparation method,

the number of cycles in the first round of PCR is 15-30, 15-20 or 15-18;
the number of cycles in the second round of PCR is 15-20 or 18-22; and/or
the number of cycles in the third round of PCR is 6-10 or 6-8.

In some embodiments of the present disclosure, in the preparation method,

the step 3) further comprises the step of: performing screening to obtain the first round of PCR products having a fragment length of less than 1000 bp;
the step 4) further comprises the step of: performing screening to obtain the second round of PCR products having a fragment length of less than 1000 bp; and/or
the step 5) further comprises the step of: performing screening to obtain the third-round PCR products having a fragment length of less than 1000 bp.

In some embodiments of the present disclosure, in the preparation method, the first round of PCR products, the second round of PCR products and/or the third round of PCR products having a fragment length of less than 1000 bp are obtained by magnetic bead screening.

In some embodiments of the present disclosure, in the preparation method, the internal reference gene is a house-keeping gene, for example, gene *ATCB*, gene *GAPDH* or gene *ApoB.*

In some embodiments of the present disclosure, in the preparation method, the retrovirus is a lentivirus or a γ-retrovirus.

Another aspect of the present disclosure relates to a DNA library prepared by the preparation method of any one in the present disclosure; wherein preferably, the DNA library is a sequencing library; more preferably, the DNA library is a sequencing library for detecting a retroviral integration site.

A still further aspect of the present disclosure relates to a method for detecting a retroviral integration site, comprising the steps of sequencing the DNA library of the present disclosure and obtaining sequencing data; wherein preferably, the sequencing is second generation sequencing.

A still further aspect of the present disclosure relates to a method for detecting a retroviral integration site, comprising the method for preparing a DNA library of any one in the present disclosure, and further comprising the step of:

6) sequencing the resulting DNA library to obtain sequencing data;
wherein preferably, the sequencing is second generation sequencing.

In some embodiments of the present disclosure, the detection method further comprises the step of:
7) performing bioinformatics analysis on the sequencing data to obtain integration site data.

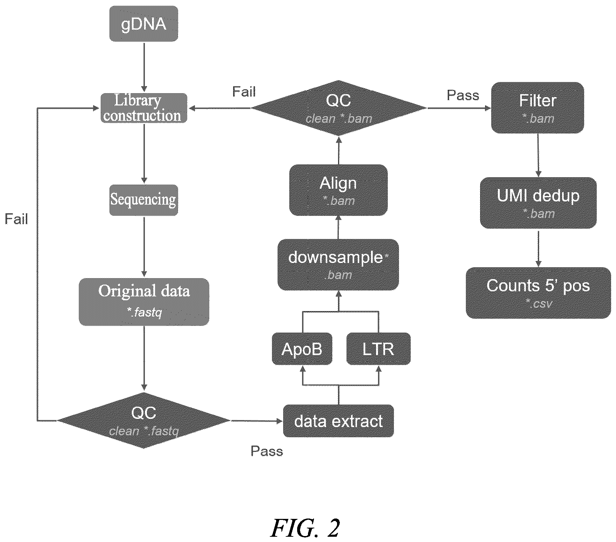

In some embodiments of the present disclosure, the process of bioinformatics analysis is as shown in FIG. 2.

In the bioinformatics analysis process of the present disclosure, a cutoff value is set to filter ultra-low frequency fragments, and the cutoff value is adjusted so that the detection results after background removal are as consistent as possible with the theoretical values/values verified by other methods such as ddPCR. False positive fragments due to PCR or sequencing errors are effectively reduced; downsample is performed on the sequencing data so that the depth of sequencing for an internal reference gene and integration site fragments remains consistent; and UMI deduplication is used to reduce the result differences caused by different fragment amplification efficiencies.

In some embodiments of the present disclosure, the detection method is a quantitative detection method, preferably an absolute quantitative detection method.

In some embodiments of the present disclosure, in the detection method, the retrovirus is a lentivirus or a $\gamma$-retrovirus.

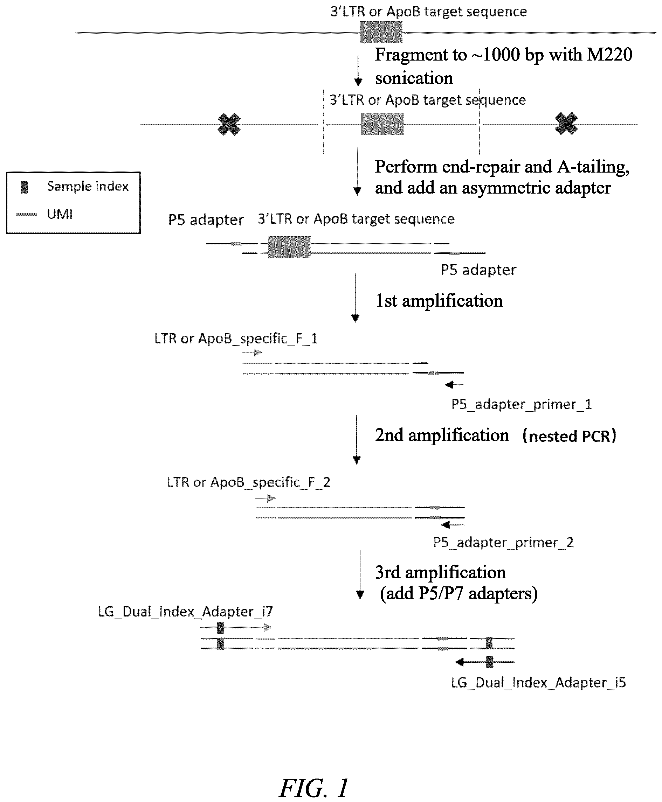

In some embodiments of the present disclosure, the process of the detection method is as shown in FIG. 1.

In a particular embodiment of the present disclosure, the general process of the method for absolute quantitative detection of a retroviral integration site is as follows:

1. a genomic DNA is sonicated into small fragments of about 1000 bp using a sonicator;
2. after end-repair and A-tailing, TA is ligated with an adapter of known sequence;
3. the ligation product is subjected to a first round of amplification with primers that specifically bind to an LTR sequence/ApoB (internal reference gene) sequence and primers that specifically bind to the adapter sequence to enrich the sequence containing the LTR/ApoB fragments in the sonicated sequences;
4. magnetic bead screening of product fragments below 1000 bp is performed, and the first round of amplification products are subjected to a second round of PCR (nested amplification) with an additional set of primers that specifically bind to the LTR sequence/ApoB (internal reference gene) sequence and primers that specifically bind to the adapter sequence to further enrich the sequence containing LTR/ApoB fragments in the sonicated sequences;
5. magnetic bead screening of product fragments below 1000 bp is performed, and the second round of PCR products are subjected to a third round of PCR amplification with primers having sequencing adapter fragments to add the sequencing adapters; and
6. magnetic bead screening of product fragments below 1000 bp is performed, and the screened products are subjected to second generation sequencing using an Illumina sequencer.

On the basis of conforming to common knowledge in the art, the above-mentioned preferred conditions can be arbitrarily combined to obtain various embodiments of the present disclosure.

Reagents and raw materials used in the present disclosure are all commercially available.

A still further aspect of the present disclosure relates to a method for preparing an engineered immune cell, comprising the step of detecting the engineered immune cell according to the detection method of any one in the present disclosure.

In some embodiments of the present disclosure, further comprised are identifying the integration sites and/or integration frequencies of retroviruses (e.g., lentiviruses or $\gamma$-retroviruses), analyzing the characteristics of genomic alterations, assessing the safety associated with lentiviral integration sites, and assessing potential risks. By means of assessment, engineered immune cells that have a low probability or no probability of developing abnormal cell behavior (for example, causing a mutation in a key gene or activating a proto-oncogene, which in turn leads to an increased risk of developing a malignant tumor) and meet relevant safety criteria can be released for subsequent scale-up culture, preservation or combination with a pharmaceutically acceptable carrier to obtain a pharmaceutical composition, etc.

In some embodiments of the present disclosure, in the method for preparing an engineered immune cell, the engineered immune cell is obtained by a method comprising the steps of:

(1) isolating immune cells;

(2) constructing a recombinant retroviral vector comprising a nucleic acid encoding a chimeric antigen receptor; and
(3) introducing the recombinant retroviral vector obtained in step (2) into the immune cells.

In particular, the method for preparing an engineered immune cell may comprises transfecting or transducing immune cells isolated from an individual such that the immune cells express desired CAR(s). Methods for preparing engineered immune cells for immunotherapy are described in, for example, WO 2014/130635, WO 2013/176916 and WO 2013/176915, which are incorporated herein by reference. Individual steps that can be used to prepare engineered immune cells are disclosed in, for example, WO 2014/039523, WO 2014/184741, WO 2014/191128, WO 2014/184744 and WO 2014/184143, which are incorporated herein by reference.

For example, provided is a method for preparing an engineered immune cell, comprising the steps of isolating an immune cell, preparing a retroviral vector encoding a CAR (chimeric antigen receptor), introducing the vector into the immune cell, etc. to obtain the engineered immune cell. The obtained engineered immune cell is subjected to the step of performing absolute quantitative detection of a retroviral integration site of any one in the present disclosure, thereby assessing whether the engineered immune cells prepared achieve the desired effect without potential safety risks. As a result, the engineered immune cells that meet the expected requirements are obtained.

In some embodiments of the present disclosure, in the method for preparing an engineered immune cell, the engineered immune cell is selected from a cytotoxic T cell, a helper T cell, a natural killer T cell, a γδ T cell, an NK cell, a macrophage, a B cell, an antigen presenting cell, a dendritic cell and a stem cell-induced immune cell.

In a particular embodiment of the present disclosure, the engineered immune cell is a CAR-T cell.

The present disclosure also relates to any one or more of items 1 to 13 selected from:

1. A method for absolute quantitative detection of a lentiviral integration site, wherein the method comprises the steps of:

1) fragmenting a genomic DNA from a lentivirus-infected cell;
2) subjecting the fragmented DNA to end-repair, A-tailing, and ligation with an adapter to obtain a ligation product, wherein the adapter is an asymmetric double-strand adapter comprising a long-strand sequence and a short-strand sequence, wherein the long-strand sequence sequentially comprises, from a 5' end to a 3' end, a fixed sequence, a random UMI sequence, and an amplification primer binding sequence, and the short-strand sequence comprises a sequence complementary to the fixed sequence;
3) subjecting the ligation product to a first round of PCR amplification with primers that specifically bind to an LTR sequence/internal reference gene sequence and primers that specifically bind to the adapter sequence to enrich the sequence containing the LTR/internal reference gene fragments in the sonicated sequences;
4) subjecting the first round of PCR products to a second round of PCR amplification with an additional set of primers that specifically bind to the LTR sequence/internal reference gene sequence and primers that specifically bind to the adapter sequence to further enrich the sequence containing the LTR/internal reference gene fragments in the sonicated sequences;
5) subjecting the second round of PCR products to a third round of PCR amplification with primers having sequencing adapter fragments to add the sequencing adapters; and
6) sequencing the screened products.

2. The method for absolute quantitative detection of item 1, wherein the fixed sequence is 14-30 bases in length; and/or the UMI sequence is a sequence NNNNNNNN of 5-9 bases in length, and the N represents base A, G, C or T.

3. The method for absolute quantitative detection of item 1 or item 2, wherein the primers that bind to the LTR sequence/internal reference gene sequence in the second round of PCR are located downstream of the primers that bind to the LTR sequence/internal reference gene sequence in the first round of PCR, and the primers that bind to the adapter sequence in the second round of PCR cover the primers that bind to the adapter sequence in the first round of PCR and extend downstream.

4. The method for absolute quantitative detection of any one of items 1 to 3, wherein the number of amplification cycles in steps 3) -5) is 36-63.

5. The method for absolute quantitative detection of any one of items 1 to 4, wherein the ratio of the number of cycles in the first round of PCR to that in the second round of PCR is (1-3) : 2, for example, 1 : 2, 3 : 2, 1 : 1 or 3 : 4;
preferably, the number of cycles in the first round of PCR and the second round of PCR is 30 and 20, or 15 and 20, or 10 and 20, or 15 and 15, respectively.

6. The method for absolute quantitative detection of any one of items 1 to 5, wherein the number of cycles in the third round of PCR is 6-13, for example, 6, 10 or 13;
preferably, the number of cycles in the first round of PCR, the second round of PCR and the third round of PCR is 15, 20 and 6, respectively.

7. The method for absolute quantitative detection of any one of items 1 to 6, wherein the 5' end of the short-strand sequence is provided with a modification that prevents primer extension, preferably an inverted dT modification fragment or a dideoxycytidine;

and/or, the internal reference gene is selected from gene *ATCB*, gene *GAPDH* and gene *ApoB*.

8. The method for absolute quantitative detection of any one of items 1 to 7, wherein the genomic DNA in step 1) is sonicated into small fragments of about 1000 bp.

9. The method for absolute quantitative detection of any one of items 1 to 8, wherein in steps 4)-6), product fragments below 1000 bp are screened for the second round of PCR, the third round of PCR and sequencing respectively.

10. The method for absolute quantitative detection of any one of items 1 to 9, wherein the sequencing in step 6) is second generation sequencing.

11. A method for preparing an engineered immune cell, wherein the method for preparing an engineered immune cell comprises the step of performing the method for absolute quantitative detection of a lentiviral integration site of any one of items 1 to 10 on the engineered immune cell.

12. The method for preparing an engineered immune cell of item 11, wherein the engineered immune cell is obtained by at least the steps of:

   (1) isolating immune cells;
   (2) preparing a lentiviral vector encoding a CAR (chimeric antigen receptor); and
   (3) introducing the vector obtained in step (2) into the immune cells.

13. The method for preparing an engineered immune cell of item 11 or item 12, wherein the engineered immune cell is selected from a cytotoxic T cell, a helper T cell, a natural killer T cell, a $\gamma\delta$ T cell, and an NK cell.

Beneficial effects of the invention

The present disclosure achieves any one or more of the following technical effects (1) to (8):

   (1) the method of the present disclosure has a sensitivity verified to reach a single site copy number of 17 copies/$\mu$g gDNA (as shown in Table 13), and is the first integration site detection method with a definite sensitivity that can be truly applied to the detection of clinical samples;
   (2) the method of the present disclosure minimizes the number of amplification cycles, thereby effectively reducing the soft-clip ratio and base deviations generated by amplification;
   (3) the bioinformatics analysis process used in the method of the present disclosure undergoes multiple rounds of adjustment and verification, resulting in the key cutoff for filtering noise;
   (4) in the method of the present disclosure, the internal reference gene is set to calibrate the actual amount of DNA in a formed library, and compared the actual amount of DNA in the formed library with the detection results of integration sites, the absolute quantification of individual integration sites (the number of copies per microgram of DNA for a certain integration site) can be realized, instead of just obtaining the ratio between the individual integration sites, and at the same time, the product of each library is within a controllable range, thereby stabilizing the operation steps in the library construction process (setting of the parameter of PCR cycle number), avoiding repeated library construction;
   (5) in the method of the present disclosure, UMI deduplication is set to effectively reduce fragment amplification errors and differences in amplification efficiency of different fragments caused during PCR;
   (6) in the present disclosure, the adapter sequence is improved, wherein a modification (inverted dT) for blocking amplification is performed on the ends of the short strand, so that the product from the spontaneous amplification with the adapter primers in the first round is greatly reduced;
   (7) the method of the present disclosure uses only sonication, PCR and magnetic bead purification, which not only avoids the preference of enzymatic cleavage, but also does not use streptavidin-labeled magnetic beads to pull down biotinylated target fragments, thereby providing advantages of simple step and low cost compared to nrLAM PCR, etc.
   (8) the present disclosure has broad application prospects, and be applied to the detection of lentiviral integration sites in all biological samples from which DNA can be effectively extracted, including, but not limited to, the detection of various cell therapy products modified with lentiviruses and clinical cell samples from patients. For example, as a quality control standard for safety, the present disclosure can be applied to QC release of CAR-T products; and when detecting clinical cell samples from patients, the present disclosure can monitor the dynamic changes in CAR-T cells at different integration sites in real time, and can be used to assess the efficacy, safety, etc. of cell therapy products.

Brief Description of the Drawings

FIG. 1: Experimental flow chart of example.

FIG. 2: Bioinformatics analysis flow chart of example.

FIG. 3A: Electropherogram exported from tapestation 4150.

FIG. 3B: Electropherogram exported from tapestation 4150.

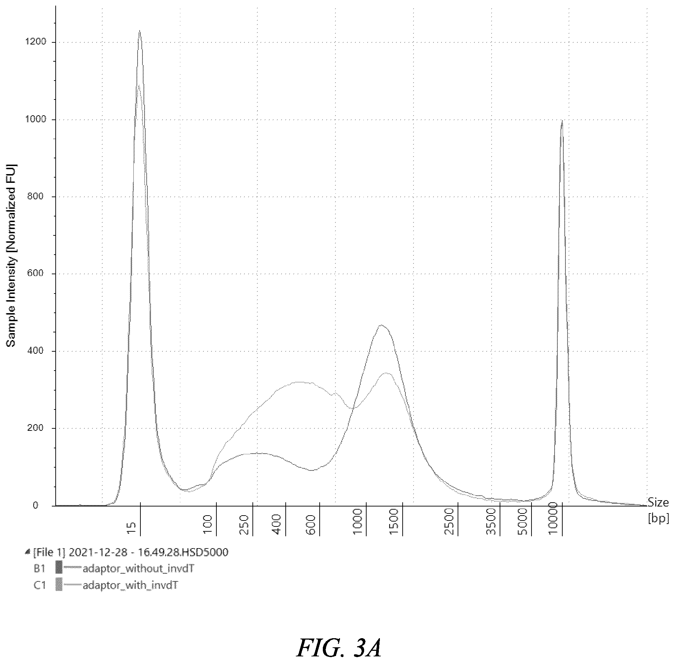

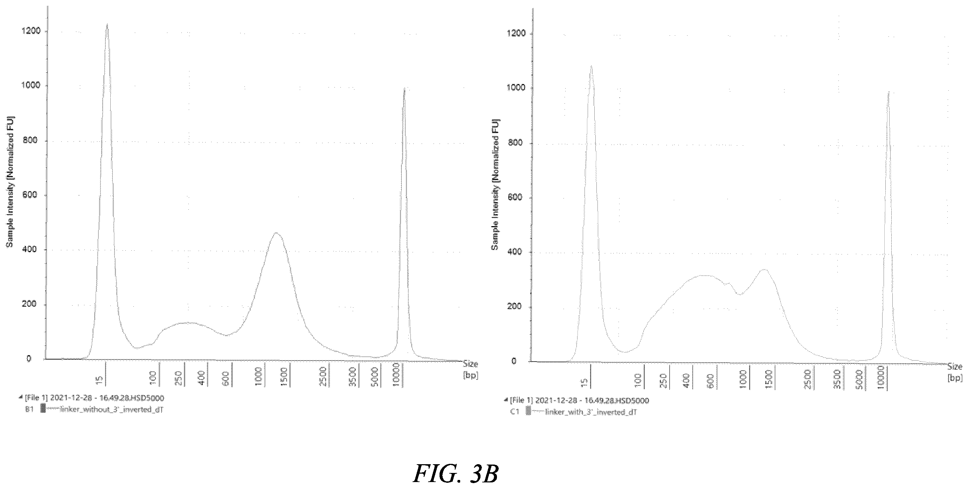

FIG. 3A and FIG. 3B show that the use of the adapter with inverted dT is better for fragment enrichment than the use of the adapter without inverted dT.

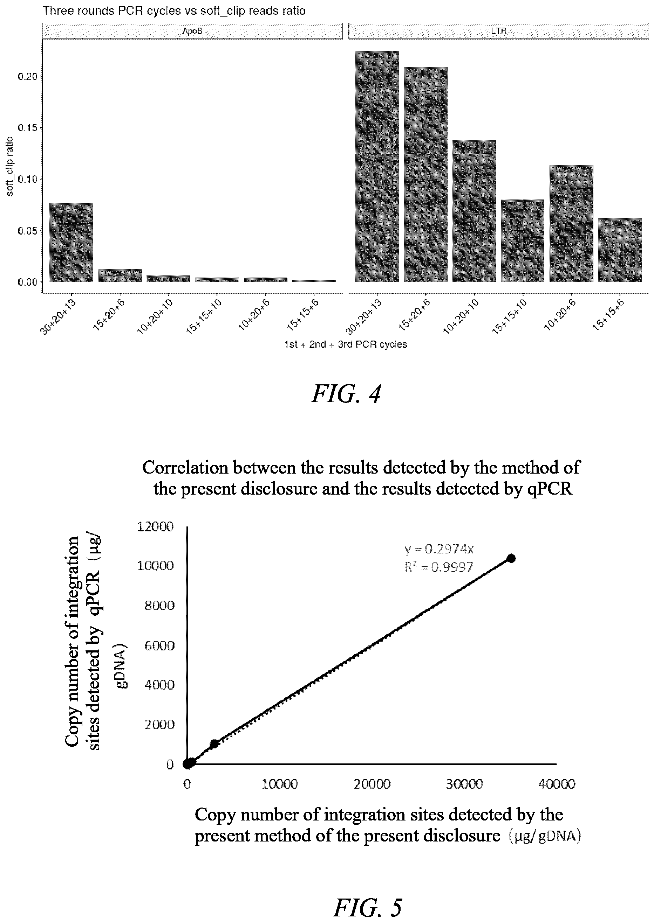

FIG. 4: The softclip ratio in the sequencing results of the libraries constructed with different cycle numbers, showing the effect of the setting of different numbers of cycles in three rounds of PCRs on the soft-clip ratio in the final library.

FIG. 5: Dot plot of linear correlation based on Table 13, showing the correlation of the detection results of the method of the present disclosure with the detection results of the qPCR method.

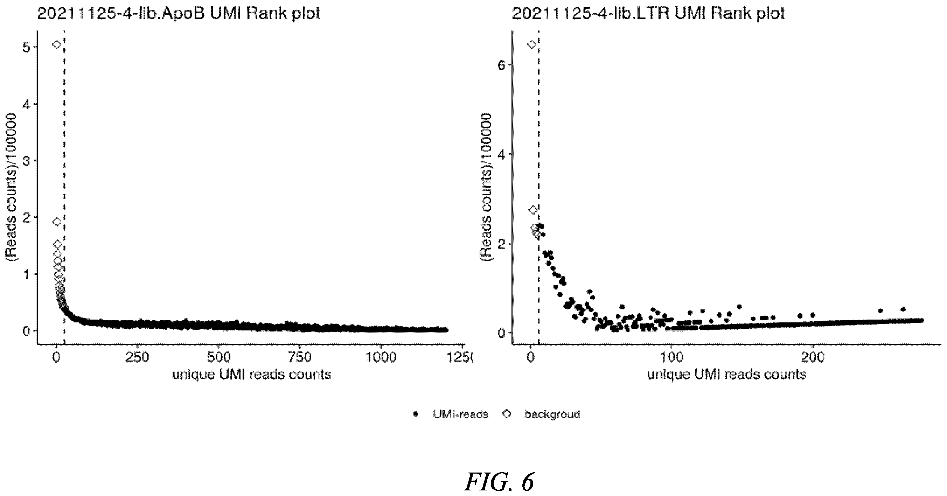

FIG. 6: Dot plot of the number of identical initial molecules vs. the detected reads counts, based on the sequencing results, showing the distribution of reads counts after library construction amplification of the initial molecules before filtering.

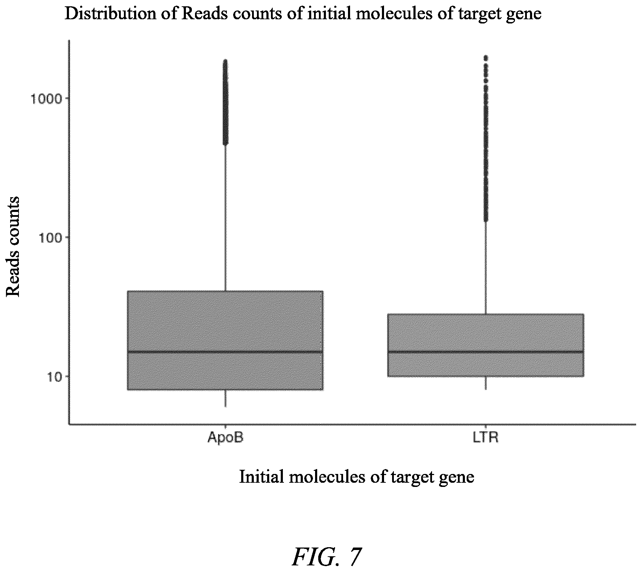

FIG. 7: Box plot of the number of initial molecules vs. the detected reads counts, showing the distribution of reads counts after library construction amplification of the initial molecules.

Detailed Description of Embodiments

The present disclosure is further described below by way of examples; however, the present disclosure is not limited to the scope of the described examples. For the experimental methods in which no specific conditions are specified in the following examples, selections are made according to conventional methods and conditions or according to the product instructions.

The test cell line gDNA for the detection of lentiviral integration sites in the present disclosure is derived from a CAR-T positive single copy cell line independently prepared and preserved in our company with a positivity rate close to 100%, verified by qPCR and ddPCR, and stored at -20 degrees Celsius (°C). CAR-T cells are themselves a source of genomic DNA, so the present disclosure is not limited to the above-mentioned cell line, and any cell from which DNA can be obtained is applicable.

The genomic DNA used in the present disclosure is derived from CART cells, but the DNA used in the method is not limited to DNA derived from CART cells. For example, the DNA can also be the international standard WHO 1st Reference Reagent 2019 for lentiviral vector integration site analysis, available from: https://www .nibsc.org/pro ducts/brm_product_catalogue/detail_page.aspx?cati d=18/144.

The test cell line gDNA for the detection of retroviral integration sites in the present disclosure is derived from two independently prepared and preserved monoclonal cell lines (cell lines 1 and 2) in our company which are isolated after γ-retrovirus transfection, and verified by ddPCR. The present disclosure is not limited to the above cell lines, and any cell from which DNA can be obtained is applicable.

Example 1: DNA fragmentation

1.1 300 to 1000 ng of genomic DNA was taken, diluted to a total volume of 130 μL using Ultrapure water, and mixed uniformly.

1.2 130 μL of the resulting solution was all added into a microTUBE AFA Fiber Snap-Cap tube.

1.3 The following program was run on a Covaris M220 to sonicate the genomic DNA:

Table 1

| Settings | Parameter |
| --- | --- |
| Temperature (°C) | 20 |
| Target Base Pair Peak (bp) | 1500 |
| Peak Incident Power (W) | 50 |
| Duty Factor (%) | 2 |
| Cycles per Burst (cpb) | 200 |
| Treatment Time (sec) | 20 |

1.4 After the sonication was completed, the sonicated products were immediately transferred to an EP tube for the next step.

1.5 To the sonicated products in the previous step were added 230 μL of (1.8×) SPRIselect beads, then the resulting mixture was mixed uniformly using a pipette, and incubated for 5 min at room temperature.

1.6 The EP tube was transferred to a magnetic stand. After the liquid was clear, 350 μL of the liquid was aspirated and discarded.

1.7 300 μL of 80% (v/v) ethanol was added, and after 30 s, the supernatant was carefully removed.

1.8 200 μL of 80% ethanol was added, and after 30 s, the supernatant was carefully removed.

1.9 The EP tube was centrifuged, and then transferred to the magnetic stand, and the residual liquid was aspirated.

1.10 Drying was performed for 2 min at room temperature, the EP tube was removed from the magnetic stand, and 50.5 μL of EB buffer was immediately added to resuspend the magnetic beads.

1.11 Incubation was performed for 5 min at room temperature, and 50 μL of the product was transferred into a PCR tube.

Example 2: End repair and purification

To 50 μL of the product was added 15 μL of End Prep Mix 4 (Vazyme N203-02), then the resulting mixture was mixed uniformly, centrifuged, and placed in a PCR instrument, and the following program was run:

Table 2

| Temperature | Time |
|---|---|
| Lid Temperature (105°C) | On |
| 20°C | 15 min |
| 65°C | 15 min |
| 4°C | Hold |

2.2 SPRIselect beads were shaken until completely resuspended.

2.3 52 μL of SPRIselect beads (0.8×) were added to 65 μL of each sample, uniformly mixed by pipetting 15 times using a tip, and incubated for 5 min at room temperature.

2.4 The PCR tube was placed on a magnetic stand until the supernatant was clear and transparent, and the supernatant was carefully removed and discarded.

2.5 200 μL of 80% ethanol was added, and after 30 s, the supernatant was carefully removed.

2.6 200 μL of 80% ethanol was added, and after 30 s, the supernatant was carefully removed.

2.7 The PCR tube was centrifuged, and transferred to the magnetic stand, and the residual liquid was aspirated.

2.8 Drying was performed for 2 min at room temperature, the EP tube was removed from the magnetic stand, and 21 μL of 1×IDTE Buffer (IDT, 11-05-01-09) was immediately added to resuspend the magnetic beads.

2.9 Incubation was performed for 5 min at room temperature, and 20 μL of the product was transferred into a PCR tube.

Example 3: Adapter annealing

3.1 DNA oligo to be annealed was formulated with NFW (Nuclease-Free Water) to a solution with a final concentration of 50 μM. Annealing Buffer for DNA Oligos (5×) (Beyotime D0251) was dissolved and mixed uniformly for later use.

3.2 The information of the specific adapters used is as shown in Table 16.

3.3 An annealing system was set up as follows:

Table 3

| Components | Volume |
|---|---|
| Nuclease-Free Water | 40 μL |
| Annealing Buffer for DNA Oligos (5×) | 20 μL |
| Adapter_F (50 μM) | 20 μL |
| Adapter_R (50 μM) | 20 μL |
| Total | 100 μL |

3.4 The following program was run on an ABI proflex PCR system instrument:

Table 4

| Temperature | Time |
| --- | --- |
| Lid Temperature (105°C) | On |
| 95°C | 2 min |
| A drop of 0.1°C every 8 seconds, to 25°C. | about 90 min |
| 4°C | Hold |

Example 4: Fragment-adapter ligation and purification

4.1 2×Rapid Ligation buffer and T4 DNA ligase (Vazyme N103) were thawed, then uniformly mixed by inverting, and put on ice for later use. The ligation reaction system was formulated according to the following table:

Table 5

| Components | Volume |
| --- | --- |
| End-repair products | 20 μL |
| 2× Rapid Ligation buffer | 30 μL |
| T4 DNA ligase | 5 μL |
| Adapter (10 μM) | 5 μL |

4.2 The system was uniformly mixed using a pipette, and centrifuged for a short time, the above-mentioned mixed system was placed in an ABI proflex PCR system instrument, and the program as shown in the following table was run:

Table 6

| Temperature | Time |
| --- | --- |
| Lid Temperature (40°C) | On |
| 30°C | 20 min |
| 4°C | Hold |

4.3 SPRIselect beads were shaken for uniformly mixing, and completely and uniformly mixed using a pipette.
4.4 48 μL of SPRIselect beads (0.8×) were pipetted into the ligation product, completely and uniformly mixed using a pipette, and incubated for 5 min at room temperature.
4.5 The EP tube was transferred to a magnetic stand. After the liquid was clear, the supernatant was carefully removed and discarded.
4.6 200 μL of 80% ethanol was added, and after 30 s, the supernatant was carefully removed.
4.7 200 μL of 80% ethanol was added, and after 30 s, the supernatant was carefully removed.
4.8 The PCR tube was centrifuged, and transferred to the magnetic stand, and the residual liquid was aspirated.
4.9 Drying was performed for 2 min at room temperature, the EP tube was removed from the magnetic stand, and 21 μL of 1×IDTE Buffer was immediately added for resuspension.
4.10 Incubation was performed for 5 min at room temperature, and 20 μL of the product was transferred into a new PCR tube.

Example 5: First round of PCR (lentivirus as an example)

5.1 The components in the Phanta Super-Fidelity DNA Polymerase (Vazyme P501) kit were thawed and then completely shaken, wherein all operations should be performed on ice.

5.2 The first round PCR reaction system was formulated as follows:

Table 7

| Components | Volume |
|---|---|
| 5× SF Buffer | 10 μL |
| LTR_F_1 & ApoB _F_1 (10 μM) | 2 μL each |
| P5-Adapter_1 (10 μM) | 4 μL |
| dNTP Mix (10 mM each) | 1 μL |
| Phanta Super-Fidelity DNA Polymerase | 1 μL |
| Template DNA (purified products with ligated adapter) | 20 μL |
| Nuclease-free water (NFW) | to 50 μL |

The information of the primers used in this step is as shown in Table 16 below.
5.3 The system was pipetted using a pipette, and centrifuged for a short time, the above-mentioned mixed system was placed in an ABI proflex PCR system instrument, and the program as shown in the following table was run:

Table 8

| Temperature | Time | Number of cycles |
|---|---|---|
| Lid Temperature (105°C) | On | / |
| 95°C | 3 min | / |
| 95°C | 10 sec | |
| 60°C | 15 sec | 15 cycles |
| 72°C | 15 sec | |
| 72°C | 5 min | / |
| 4°C | Hold | / |

5.4 SPRIselect beads were shaken until completely resuspended.
5.5 30 μL of SPRIselect beads (0.6×) were pipetted into each sample, uniformly mixed by pipetting 15 times using a tip, and incubated for 5 min at room temperature.
5.6 The PCR tube was placed on a magnetic stand until the supernatant was clear and transparent, and then the supernatant was transferred to a new PCR tube.
5.7 10 μL of SPRIselect beads (0.8×) were added to each sample, uniformly mixed by pipetting 15 times using a tip, and incubated for 5 min at room temperature.
5.8 The PCR tube was placed on a magnetic stand until the supernatant was clear and transparent, and the supernatant was carefully removed.
5.9 200 μL of 80% ethanol was pipetted into each tube, and let to stand for 30 s, and the ethanol liquid was carefully removed and discarded.
5.10 200 μL of 80% ethanol was pipetted into each tube, and let to stand for 30 s, and the ethanol liquid was carefully removed and discarded.
5.11 The PCR tube was centrifuged instantaneously, and was placed on the magnetic stand, the residual ethanol was removed and discarded, and drying was performed for 2 min at room temperature.
5.12 The PCR tube was taken from the magnetic stand, 21 μL of 1×IDTE Buffer was added and uniformly mixed by pipetting 15 times using a tip, and incubated for 2 min at room temperature.
5.13 The PCR tube was placed on a magnetic stand until the supernatant was clear and transparent.
5.14 20 μL of the sample was transferred into a new tube, and the sample was stored at 4°C for 72 h or at -20°C for a long period of time, or directly used for the next experiment.

Example 6: Second round of PCR (lentivirus as an example)

6.1 The components in the kit were thawed and then completely shaken, wherein all operations should be performed on ice.

6.2 The second round PCR reaction system was formulated as follows:

Table 9

| Components | Volume |
|---|---|
| 5× SF Buffer | 10 μL |
| dNTP Mix (10 mM each) | 1 μL |
| Phanta Super-Fidelity DNA Polymerase | 1 μL |
| P5-Adapter_2 (10 μM) | 4 μL |
| LTR_F_2 & ApoB_F_2 (10 μM) | 2 μL each |
| Temple DNA (first round of PCR products) | 17 μL |
| NFW | to 50 μL |

6.3 The information of the primers used in this step is as shown in Table 16 below.

6.4 The system was pipetted using a pipette, and centrifuged for a short time, the above-mentioned mixed system was placed in an ABI proflex PCR system instrument, and the program as shown in the following table was run:

Table 10

| Temperature | Time | Number of cycles |
|---|---|---|
| Lid Temperature (105°C) | On | / |
| 95°C | 3 min | / |
| 95°C | 10 sec | |
| 60°C | 15 sec | 20 cycles |
| 72°C | 15 sec | |
| 72°C | 5 min | / |
| 4°C | Hold | / |

6.5 Steps 5.4-5.14 were repeated.

Example 7: Third round of PCR

7.1 The components in the kit were thawed and then completely shaken, wherein all operations should be performed on ice.

7.2 The third round PCR reaction system was formulated as follows:

Table 11

| Components | Volume |
|---|---|
| 5× SF Buffer | 10 μL |
| P5-Adapter_3 (10 μM) | 2 μL |
| P7-Adapter_3 (10 μM) | 2 μL |
| dNTP Mix (10 mM each) | 1 μL |
| Phanta Super-Fidelity DNA Polymerase | 1 μL |
| Temple DNA (second round of PCR products) | 25 ng |
| NFW | to 50 μL |

The information of the primers used in this step is as shown in Table 16 below.

7.3 The system was pipetted using a pipette, and centrifuged for a short time, the above-mentioned mixed system was placed in an ABI proflex PCR system instrument, and the program as shown in the following table was run:

Table 12

| Temperature | Time | Number of cycles |
|---|---|---|
| Lid Temperature (105°C) | On | / |
| 95°C | 3 min | / |
| 95°C | 10 sec | |
| 60°C | 15 sec | 6 cycles |
| 72°C | 15 sec | |
| 72°C | 5 min | / |
| 4°C | Hold | / |

7.4 Steps 5.4-5.13 were repeated.

7.5 20 μL of the sample was transferred into a new tube. The concentration of the purified product was detected using Qubit, and the size of fragments was detected using Tapestation 4150. The library can subsequently be put directly into Illumina sequencer for sequencing.

Example 8: Bioinformatics analysis process (lentivirus as an example)

The data analysis process involved in the present method is as follows:

1. fastqc was used to view quality control information on the raw fastq data for sequencing, and fastp was used to clean the sequencing data, wherein only reads having the sequencing quality value greater than 25, the reads length greater than 80, and the N bases less than or equal to 3, and at most 5% of the bases that fail to correctly match were retained; and fastqc was used again for re-confirmation of quality control information on the cleaned fastq data.

2. As for the cleaned fastq data, the first 8 bases of R1 sequencing data were extracted as UMI using umi_tools and added to the coordinates of reads, and the UMI sequence was mapped into R2 sequencing data according to the coordinates, then the data was split according to the primer sequences of LTR/ApoB with the error tolerance set to no more than 2 bases.

3. 3000000 reads were randomly extracted from the split ApoB fastq file and the sampling ratio was calculated, followed by the extraction of LTR reads at the same ratio.

4. The sampled ApoB/LTR reads were aligned to the human genome using BWA, and the reference sequence was GRCh38. The original bam file was further filtered to remove the reads with soft clip base number greater than 15, alignment quality less than 20, reads length less than 50 and hard clip contained. Then, according to the sequences of UMI and reads, the unique umi reads counts was counted, then alignment and deduplication were performed, after which the median was calculated, with reads having the umi reads counts less than 1% of the median removed as a background.

5. Double-end deduplication of UMI using umi_tools was performed on the cleaned bam file, the alignment quality was set to be greater than 20, chimeric-pairs and unpaired-reads were discarded, and then all inverse sequencing reads were extracted using flag.

6. bedtools was used to count and report the position of the 5' end of inverse sequencing reads.

Experimental results:

Inverted dT modifications on the short strand of the adapter can avoid or reduce non-specific amplification in the PCR reaction. According to the experimental methods described in the present disclosure, ligation and repair were performed on the sonicated DNA using adapters with inverted dT and adapters without inverted dT, respectively, the first round of PCR was then performed, followed by the purification using $0.8\times$ magnetic beads, then the second round of PCR was performed, and the fragment size and distribution analysis were performed on the samples in the constructed library using Tapestation 4150 automated electrophoresis instrument. The results are as shown in FIG. 3A and FIG. 3B. The results show that the proportion of target fragments (< 1000 bp) when using of the adapters with inverted dT is higher than that when using the adapters without inverted dT.

The number of amplification cycles was minimized, thereby effectively reducing the soft-clip ratio and base deviations generated by amplification. By adjusting the number of cycles in each of the three rounds of PCR, the soft-clip ratio was counted after extracting the reads of the target gene by the bioinformatics analysis process of the present disclosure as described above. The results show that the soft-clip ratio decreases as the total number of cycles decreases, as shown in FIG. 4.

By introducing the primer set of internal reference gene hApoB while determining the lentiviral integration site, the amount of the sonicated fragments introduced can be obtained from the internal reference gene so as to achieve the absolute quantification of the copy number of each integration site and determination of the sensitivity of the method.

Using this method, cell lines with known insertion sites that were gradiently diluted were detected. The results are as shown in Table 13 and FIG. 5, wherein the copy number detection result (IS/µg gDNA) is calculated as follows:

$$\text{IS copy number} / \text{µg gDNA} = \frac{\text{IS reads counts}}{\text{ApoB reads counts} \times 3.3 \times 10^{-6} \text{µg genome}}$$

Table 13: Sensitivity results of the methods of the present disclosure

| Sample | reads counts of integration site detected | reads counts of internal reference gene site detected | Detection results of the method of the present disclosure (IS copy number/µg gDNA) | Detection results of qPCR (IS copy umber/µg gDNA)) |
|---|---|---|---|---|
| | chr17:66493342, forward_chain | chr2:21005153, reverse_chain | | |
| Normal human PBMC | 0 | 31680 | 0 | 7 |
| 0.01%_Cell line 1 | 10 | 28815 | 105 | 17 |
| 0.02%_Cell line 1 | 4 | 31272 | 39 | 33 |
| 0.05%_Cell line 1 | 8 | 32344 | 75 | 62 |
| 0.1%_Cell line 1 | 48 | 29355 | 496 | 107 |
| 1%_Cell line 1 | 272 | 28003 | 2943 | 1050 |
| 10%_Cell line 1 | 3780 | 32706 | 35023 | 10401 |

The bioinformatics analysis was performed for further optimization, wherein background noise was effectively filtered by setting cutoff values. Taking 20211125-4-lib as an example, 10 ng of Jurkat clone10 P32 (a single copy cell line verified by ddPCR, with a copy number of LTR : ApoB = 1 : 2) and 990 ng of normal human PBMC were selected to prepare a simulation sample at a mass ratio of 1%, that is, the theoretical copy number ratio of LTR : ApoB was 0.5%. The initial molecules in the original bam alignment file were counted by library construction, sequencing and analysis using the method as described above, the median was calculated for the number of initial molecules, and the reads counts corresponding to the initial molecules which was less than 1% of the median was set as background. The plot is as follows (FIG. 6), and the results obtained with filtering are significantly closer to the theoretical values than those obtained without filtering (Table 14).

Table 14: Effects of setting of cutoff values to filter ultra-low frequency reads on result readout

| Gene | Original reads counts | Reads counts after Downsample | Deduplicati on without background removal | Deduplicati on with background removal | Theoreti cal value |
|---|---|---|---|---|---|
| LTR | 240139 | 43231 | 1773 | 144 | / |
| ApoB | 16581336 | 3000000 | 191175 | 30816 | / |
| LTR/ ApoB | 1.45% | 1.44% | 0.93% | 0.47% | 0.50% |

UMI deduplication is set to effectively reduce fragment amplification errors and differences in amplification efficiency of different fragments caused during PCR. Statistics on the distribution of reads counts for the initial molecules of the target gene in 20211125-4-lib was performed (such as FIG. 7). The results show that the distribution of reads counts after amplification of different initial molecules varies from a few to more than 1000, which will greatly interfere with the results in the absence of deduplication.

With the above optimization, the method of the present disclosure can detect integration sites with single site copy numbers greater than or equal to more than 17 copies/$\mu$g gDNA in a sample, while maintaining good linearity above 107 copies/$\mu$g gDNA (Table 14). It can therefore be truly applied in the follow-up detection of clinical samples of genetically modified cell therapy based on lentiviral transfection technology. The following are examples of applications where all detection sites can be verified by ddPCR with 100% accuracy after the detection on different cell lines using the present method. The results are as shown in Table 15.

Table 15: Detection accuracy of the method of the present disclosure

| Integration sites detected | | | Proportion of integration sites detected (IS/ApoB) | | | | | | Notes |
| --- | --- | --- | --- | --- | --- | --- | --- | --- | --- |
| | | | Detection values of the method of the present disclosure | | | Detection values of ddPCR | | | |
| | | | Cell line 1 | Cell line 2 | Cell line 3 | Cell line 1 | Cell line 2 | Cell line 3 | |
| chr17 | 66493342 | forward_chain | 64.92% | 0 | 0 | 56% | NA | NA | IS-1 |
| chr9 | 135872089 | forward_chain | 0 | 41.22% | 0 | NA | 55% | NA | IS-2 |
| chr17 | 42231436 | reverse_chain | 0 | 71.88% | 0 | NA | 65% | NA | IS-3 |
| chr2 | 99457506 | reverse_chain | 0 | 0.30% | 0 | NA | 0.26% | NA | IS-4 |
| chr12 | 45871567 | reverse_chain | 0 | 0.26% | 0 | NA | 0.28% | NA | IS-5 |
| chr6 | 73520142 | forward_chain | 0 | 0 | 60.33% | NA | NA | 54.70% | IS-6 |
| chr18 | 23830594 | forward_chain | 0 | 0 | 7.16% | NA | NA | 31% | IS-7 |
| chrl | 180491177 | reverse_chain | 0 | 0 | 24.53% | NA | NA | 57.60% | IS-8 |

Table 16: Primer sequences involved in the present disclosure

| Primer name | Application steps | Sequences of adapters | Manufactu rer |
| --- | --- | --- | --- |
| Adapter_L | Asymmetric adapter se- quences | ACACTCTTTCCCTACACGACGCTCTTCCGAT CTNNNNNNNNGATCTGACTGTCTCTACCAG *T (SEQ ID NO: 1) | GenScript |
| Adapter_S | | /phos/CTGGTAGAGACAGTCAGATC-invdT (SEQ ID NO: 2) | GenScript |
| P5-Adapter_1 | Primers for first round of PCR | ACACTCTTTCCCTACACGAC (SEQ ID NO: 3) | GenScript |
| LTR_F_1 | | TTGCCTTGAGTGCTTCAAGT (SEQ ID NO: 4) | GenScript |
| ApoB _F_1 | | CACGCTTTGAGGTAGACTCT (SEQ ID NO: 5) | GenScript |

(continued)

| Primer name | Application steps | Sequences of adapters | Manufactu rer |
|---|---|---|---|
| P5-Adapter_2 | Primers for second round of PCR | ACACTCTTTCCCTACACGACGCTCTTCCGATCT (SEQ ID NO: 6) | GenScript |
| LTR_F_2 | | GTGACTGGAGTTCAGACGTGTGCTCTTCCGATCT-AGTGTGTGCCCGTCTGTTGT (SEQ ID NO: 7) | GenScript |
| ApoB_F_2 | | GTGACTGGAGTTCAGACGTGTGCTCTTCCGATCTCCCGTGTATAATGCCACTTG (SEQ ID NO: 8) | GenScript |
| P5-Adapter_3 | Primers for third round of PCR | AATGATACGGCGACCACCGAGATCTACACNNNNNNNNACACTCTTTCCCTACACGAC (SEQ ID NO: 9) | GenScript |
| P7-Adapter_3 | | CAAGCAGAAGACGGCATACGAGATNNNNNNNNGTGACTGGAGTTCAGACGTGT (SEQ ID NO: 10) | GenScript |
| /phos/: Phosphorylation modification; invdT: Inverted dT modification; *: Thio modification (T); N: Random bases, A, T, C or G. | | | |

Example 9: Detection of γ-retroviral integration sites

In addition to being applicable to the detection of integration sites of lentiviruses (or vectors), the inventors have changed the primers in the first and second rounds to be specific primers for the LTR of γ-retroviruses (such as MSCV/MoMLV), demonstrating that the method is equally applicable to the detection of integration sites of γ-retroviruses (or vectors).

1. Accuracy experiment:

Using the library construction and analysis process of the present patent, primers in the first and second rounds were changed to be specific primers (Rvbd _F_1_3 and Rvbd _F_2_3 in Table 20) for the LTR of γ-retrovirus, and two monoclonal cell lines (cell lines 1 and 2) isolated after retrovirus transfection were subjected to RIS library construction and analysis to obtain integration site information, combinations of specific primers/probes were set according to the integration site information, and verification was performed by ddPCR. The results are as shown below:

Table 17: Test of detection rate of RIS detection method

| Detected integration site (information about name and position) | | | Proportion of integration sites detected (RIS/ApoB) | | | | | | Notes |
|---|---|---|---|---|---|---|---|---|---|
| | | | Detection results by NGS | | | Detection results by ddPCR | | | |
| | | | Cell line 1 | Cell line 2 | Negative sample (PBMC) | Cell line 1 | Cell line 2 | Negative sample (PBMC) | |
| chr7 | 50338098 | forward_chain | 40.2% | 0 | 0 | 58.7% | NA | 0 | RIS-1 |
| chr12 | 101776260 | forward_chain | 27.6% | 0 | 0 | 51.4% | NA | 0 | RIS-2 |
| chr7 | 157139873 | reverse_chain | 133.6% | 0 | 0 | 56.3% | NA | 0 | RIS-3 |

(continued)

| Detected integration site (information about name and position) | | | Proportion of integration sites detected (RIS/ApoB) | | | | | | Notes |
|---|---|---|---|---|---|---|---|---|---|
| | | | Detection results by NGS | | | Detection results by ddPCR | | | |
| | | | Cell line 1 | Cell line 2 | Negative sample (PBMC) | Cell line 1 | Cell line 2 | Negative sample (PBMC) | |
| chr9 | 35618778 | reverse_chain | 151.4% | 0 | 0 | 59.6% | NA | 0 | RIS-4 |
| chr22 | 24604445 | forward_chain | 0 | 192.7% | 0 | NA | 59.8% | 0 | RIS-5 |
| chr20 | 60273580 | forward_chain | 0 | 120.7% | 0 | NA | 59.9% | 0 | RIS-6 |
| chr20 | 25091052 | forward_chain | 0 | 109.6% | 0 | NA | 60.3% | 0 | RIS-7 |
| chr5 | 77086510 | reverse_chain | 0 | 26.9% | 0 | NA | 53.1% | 0 | RIS-8 |
| chr15 | 78044944 | reverse_chain | 0 | 25.8% | 0 | NA | 59.3% | 0 | RIS-9 |
| chr16 | 10385165 | reverse_chain | 0 | 21.3% | 0 | NA | 58.3% | 0 | RIS-10 |

As can be seen from the comparison between the results detected by NGS and the results detected by ddPCR, the negative conformity rate and the positive conformity rate obtained using the NGS detection process of the present patent are both 100%, that is, the method can specifically detect RIS sites, with ddPCR as a reference.

2. Sensitivity experiment:

Cell line 1 and cell line 2 were mixed at different ratios, wherein the DNA of cell line 2, as background DNA, was mixed with 1% and 0.1% of the DNA of cell line 1, respectively, and similarly, the DNA of cell line 1, as background DNA, was mixed with 1% and 0.1% of the DNA of cell line 2, so as to prepare sensitivity samples 1, 2, 3 and 4, RIS library construction was performed, bioinformatics analysis was performed after sequencing to obtain the information of the proportion of RIS sites, and the obtained information was compared with theoretical values. The results are as shown in the table below:

Table 18: Sensitivity test of RIS detection method

| Integration sites | Proportion of integration sites detected (RIS/ALL) | | | | | | | |
|---|---|---|---|---|---|---|---|---|
| | Detection results by NGS | Theoretical value | Detection results by NGS | Theoretical value | Detection results by NGS | Theoretical value | Detection results by NGS | Theoretical value |
| | Sensitivity sample 1 (cell line 1 : cell line 2 = 1 : 99) | | Sensitivity sample 2 (cell line 1 : cell line 2 = 1 : 999) | | Sensitivity sample 3 (cell line 1 : cell line 2 = 99 : 1) | | Sensitivity sample 4 (cell line 1 : cell line 2 = 999 : 1) | |
| RIS-1 | 0.065% | 0.167% | 0.00160% | 0.0167% | 8.4% | 24.6% | 9.0% | 25.0% |
| RIS-2 | 0.049% | 0.167% | 0.00160% | 0.0167% | 5.0% | 24.6% | 5.8% | 25.0% |
| RIS-3 | 0.141% | 0.167% | 0.00801% | 0.0167% | 36.4% | 24.6% | 38.0% | 25.0% |
| RIS-4 | 0.172% | 0.167% | 0.00640% | 0.0167% | 49.3% | 24.6% | 47.1% | 25.0% |
| RIS-5 | 34.8% | 16.6% | 36.6% | 16.7% | 0.320% | 0.249% | 0.0497% | 0.025% |
| RIS-6 | 24.8% | 16.6% | 25.3% | 16.7% | 0.263% | 0.249% | 0.0097% | 0.025% |
| RIS-7 | 21.0% | 16.6% | 21.5% | 16.7% | 0.175% | 0.249% | 0.0218% | 0.025% |
| RIS-8 | 6.2% | 16.6% | 5.6% | 16.7% | 0.048% | 0.249% | UD | 0.025% |
| RIS-9 | 6.5% | 16.6% | 5.7% | 16.7% | 0.068% | 0.249% | 0.0061% | 0.025% |
| RIS-10 | 6.2% | 16.6% | 5.3% | 16.7% | 0.091% | 0.249% | 0.0036% | 0.025% |
| UD: Not detected | | | | | | | | |

The results show that the detection rate is 100% (10/10) when the sensitivity (RIS/ALL) of the present method is at a theoretical ratio of 0.167% to 0.249%, and the detection rate is 90% (9/10) when the sensitivity of the present method is at a theoretical ratio of 0.0167% to 0.025%. The sensitivity (RIS/ALL) of the present method was preliminarily determined to be below 0.167%.

Table 19: Sources of samples for RIS detection

|  | Sources | VCN/Cell | Number of cell s | Description |
|---|---|---|---|---|
| Cell line 1 | Legendbiotech | about 4 | 1E6 | Retrovirus-transfected Jurkat cell line |
| Cell line 2 | Legendbiotech | about 6 | 1E6 | Retrovirus-transfected Jurkat cell line |

Table 20: Primer sequence

| Primer name | Application steps | Sequences of adapters | Manufact urer |
|---|---|---|---|
| Adapter_L | Asymmetric adapter se-quences | ACACTCTTTCCCTACACGACGCTCTTCCGATC TNNNNNNNNGATCTGACTGTCTCTACCAG*T (SEQ ID NO: 1) | GenScript |
| Adapter_S | | /phos/CTGGTAGAGACAGTCAGATC-invdT (SEQ ID NO: 2) | GenScript |
| P5-Adapter_1 | Primers for first round of PCR | ACACTCTTTCCCTACACGAC (SEQ ID NO: 3) | GenScript |
| Rvbd _F_1_3 | | ACTTGTGGTCTCGCTGTTCCT (SEQ ID NO: 11) | GenScript |
| ApoB _F_1 | | CACGCTTTGAGGTAGACTCT (SEQ ID NO: 5) | GenScript |
| P5-Adapter_2 | Primers for sec-ond round of PCR | ACACTCTTTCCCTACACGACGCTCTTCCGATC T (SEQ ID NO: 6) | GenScript |
| Rvbd _F_2_3 | | GTGACTGGAGTTCAGACGTGTGCTCTTCCGA TCT-TGGGAGGGTCTCCTCTGAGT (SEQ ID NO: 12) | GenScript |
| ApoB_F_2 | | GTGACTGGAGTTCAGACGTGTGCTCTTCCGA TCTCCCGTGTATAATGCCACTTG (SEQ ID NO: 13) | GenScript |
| P5-Adapter_3 | Primers for third round of PCR | AATGATACGGCGACCACCGAGATCTACACNN NNNNNACACTCTTTCCCTACACGAC (SEQ ID NO: 9) | GenScript |
| P7-Adapter_3 | | CAAGCAGAAGACGGCATACGAGATNNNNNN NNGTGACTGGAGTTCAGACGTGT (SEQ ID NO: 10) | GenScript |
| /phos/: Phosphorylation modification; invdT: Inverted dT modification; *: Thio modification (T); N: Random bases, A, T, C or G. | | | |

It can be foreseen from the above examples that for the integration sites of viral/non-viral vectors for genomic integration on the basis of fixed integration sequences (such as long terminal repeats (LTRs) of retroviruses and inverted terminal repeats (ITRs) of adeno-associated viruses), the detection of integration sites of a variety of viral/non-viral vectors can be realized in conjunction with the method of the present disclosure by designing specific primers for this fixed integration sequence.

Although the specific embodiments of the present disclosure have been described in detail, it will be understood by a person skilled in the art that: according to all the teachings disclosed, various modifications and replacements can be made to those details, and these changes are within the scope of the protection of the present disclosure. The full scope of the present disclosure is given by the appended claims and any equivalents thereof.

## Claims

1. A method for preparing a DNA library, comprising the steps of:

   1) fragmenting a genomic DNA from a retrovirus-infected cell to obtain DNA fragments;
   2) subjecting the DNA fragments to end-repair, A-tailing, and ligation with an adapter to obtain a ligation product, wherein the adapter is an asymmetric double-strand adapter comprising a long-strand sequence and a short-strand sequence, wherein the long-strand sequence sequentially comprises, from a 5' end to a 3' end, a fixed sequence, a random UMI sequence, and an amplification primer binding sequence, and the short-strand sequence comprises a sequence complementary to the fixed sequence;
   3) performing a first round of PCR on the ligation product with primers for an LTR sequence, primers for an internal reference gene sequence, and primers for an adapter sequence to obtain a first round of PCR products;
   4) performing a second round of PCR on the first round of PCR products with primers for the LTR sequence, primers for the internal reference gene sequence, and primers for the adapter sequence which are different from those in step 3) to obtain a second round of PCR products; and
   5) performing a third round of PCR on the second round of PCR products with primers having sequencing adapters to obtain a third round of PCR products as a DNA library.

2. The preparation method according to claim 1, wherein in step 1), the DNA fragment is 500-1500 bp, 800-1200 bp, 900-1100 bp or 1000 bp in length.

3. The preparation method according to claim 1 or 2, wherein in step 2), the fixed sequence is 14-30 bases in length; and/or
   the random UMI sequence is 5-12 bases in length, wherein each base is independently A, G, C, or T; preferably, the random UMI sequence is 5-9 bases in length.

4. The preparation method according to any one of claims 1 to 3, wherein in step 2), the 5' end of the short-strand sequence is provided with a modification that prevents primer extension, preferably an inverted dT modification or a dideoxycytidine modification.

5. The preparation method according to any one of claims 1 to 4, wherein in step 4):

   the primers for the LTR sequence in the second round of PCR are located downstream of the primers for the LTR sequence in the first round of PCR,
   the primers for the internal reference gene sequence in the second round of PCR are located downstream of the primers for the internal reference gene sequence in the first round of PCR, and
   the primers for the adapter sequence in the second round of PCR cover the primers for the adapter sequence in the first round of PCR and extend downstream.

6. The preparation method according to any one of claims 1 to 5, wherein the total number of amplification cycles in the first round of PCR, the second round of PCR and the third round of PCR is 36-63, preferably 36-45.

7. The preparation method according to any one of claims 1 to 6, wherein the ratio of the number of cycles in the first round of PCR to that in the second round of PCR is (1-3) : 2, for example, 1 : 2, 3 : 2, 1 : 1 or 3 : 4;

   preferably, the number of cycles in the first round of PCR and the second round of PCR is 30 and 20, 15 and 20, 10 and 20, or 15 and 15, respectively;
   preferably, the number of cycles in the third round of PCR is 6-13, for example, 6, 10 or 13;
   preferably, the number of cycles in the first round of PCR, the second round of PCR and the third round of PCR is 15, 20 and 6, respectively, 10, 20 and 6, respectively, or 15, 15 and 6, respectively;
   preferably, the number of cycles in the first round of PCR is 15-18, the number of cycles in the second round of PCR is 18-22, and the number of cycles in the third round of PCR is 6-8.

8. The preparation method according to any one of claims 1 to 7, wherein the step 3) further comprises the step of: performing screening to obtain the first round of PCR products having a fragment length of less than 1000 bp;

the step 4) further comprises the step of: performing screening to obtain the second round of PCR products having a fragment length of less than 1000 bp; and/or
the step 5) further comprises the step of: performing screening to obtain the third-round PCR products having a fragment length of less than 1000 bp.

9. The preparation method according to any one of claims 1 to 8, wherein the retrovirus is a lentivirus or a γ-retrovirus.

10. The preparation method according to any one of claims 1 to 9, wherein the internal reference gene is a house-keeping gene, for example, gene *ATCB*, gene *GAPDH* or gene *ApoB*.

11. A DNA library prepared by the preparation method according to any one of claims 1 to 10, wherein preferably, the DNA library is a sequencing library; more preferably, the DNA library is a sequencing library for detecting a retroviral integration site.

12. A method for detecting a retroviral integration site, comprising the method for preparing a DNA library according to any one of claims 1 to 10, and further comprising the step of:

6) sequencing the resulting DNA library to obtain sequencing data;
wherein preferably, the sequencing is second generation sequencing.

13. The detection method according to claim 12, further comprising the step of:
7) performing bioinformatics analysis on the sequencing data to obtain integration site data.

14. The detection method according to claim 12 or 13, wherein the detection method is a quantitative detection method, preferably an absolute quantitative detection method.

15. The detection method according to any one of claims 12 to 14, wherein the retrovirus is a lentivirus or a γ-retrovirus.

16. A method for preparing an engineered immune cell, comprising the step of detecting the engineered immune cell according to the detection method according to any one of claims 12 to 15.

17. The method for preparing an engineered immune cell according to claim 16, wherein the engineered immune cell is obtained by a method comprising the steps of:

(1) isolating immune cells;
(2) constructing a recombinant retroviral vector comprising a nucleic acid encoding a chimeric antigen receptor; and
(3) introducing the recombinant retroviral vector obtained in step (2) into the immune cells.

18. The method for preparing an engineered immune cell according to claim 16 or 17, wherein the engineered immune cell is selected from a cytotoxic T cell, a helper T cell, a natural killer T cell, a γδ T cell, an NK cell, a macrophage, a B cell, an antigen presenting cell, a dendritic cell and a stem cell-induced immune cell.

3'LTR or ApoB target sequence

Fragment to ~1000 bp with M220
sonication

3'LTR or ApoB target sequence

Perform end-repair and A-tailing,
and add an asymmetric adapter

Sample index

UMI

P5 adapter   3'LTR or ApoB target sequence

P5 adapter

1st amplification

LTR or ApoB_specific_F_1

P5_adapter_primer_1

2nd amplification   (nested PCR)

LTR or ApoB_specific_F_2

P5_adapter_primer_2

3rd amplification
(add P5/P7 adapters)

LG_Dual_Index_Adapter_i7

LG_Dual_Index_Adapter_i5

*FIG. 1*

*FIG. 2*

*FIG. 3A*

*FIG. 3B*

Three rounds PCR cycles vs soft_clip reads ratio

FIG. 4

Correlation between the results detected by the method of the present disclosure and the results detected by qPCR

$y = 0.2974x$
$R^2 = 0.9997$

Copy number of integration sites detected by the present method of the present disclosure (μg/gDNA)

FIG. 5

FIG. 6

Distribution of Reads counts of initial molecules of target gene

*FIG. 7*

<table>
<tr><td colspan="2" align="center">**INTERNATIONAL SEARCH REPORT**</td><td>International application No.<br>**PCT/CN2023/083705**</td></tr>
</table>

| **A.** | **CLASSIFICATION OF SUBJECT MATTER** |
|---|---|

C12Q1/6806(2018.01)i; C12Q1/6851(2018.01)i; C12Q1/6855(2018.01)i; C40B70/00(2006.01)i; C12Q1/6869(2018.01)n

According to International Patent Classification (IPC) or to both national classification and IPC

| **B.** | **FIELDS SEARCHED** |
|---|---|

Minimum documentation searched (classification system followed by classification symbols)

IPC: C12Q1/-; C40B70/00

Documentation searched other than minimum documentation to the extent that such documents are included in the fields searched

Electronic data base consulted during the international search (name of data base and, where practicable, search terms used)

CJFD, CNTXT, CNABS, ENTXTC, WEB OF SCIENCE: 文库, 接头, 内参基因, 绝对定量, 病毒, 扩增, 短链, 封闭, 双脱氧胞苷, library, adapter, anchor, internal reference, inner reference, reference gene, ATCB, GAPDH, ApoB, absolute quantitation, virus, amplif+, short chain, block+, ddC, inverted dT, invdT, LTR

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| Y | CN 113046835 A (SHENZHEN BGI LIFE SCIENCES RESEARCH INSTITUTE) 29 June 2021 (2021-06-29)<br>    description, paragraphs 5-25 and 46, and figure 1 | 1-18 |
| Y | 董恩妮等 (DONG, Enni et al.). "实时荧光定量PCR内参基因的选择 (The Selection of Reference Gene in Real-Time Quantitative Reverse Transcription PCR)"<br>中国畜牧杂志 (Chinese Journal of Animal Science),<br>Vol. 49, No. 11, 10 June 2013 (2013-06-10), 92-96<br>ISSN: 0258-7033,<br>    page 92, left-hand column, paragraph 1-page 93, left-hand column, paragraph 1, and table 1 | 1-18 |
| Y | CN 112553337 A (HANGZHOU CHOSEN MEDICAL-EXAMINING LABORATORY CO., LTD.) 26 March 2021 (2021-03-26)<br>    claim 1 | 1-18 |
| Y | CN 101230490 B (BEIJING FORESTRY UNIVERSITY) 30 January 2013 (2013-01-30)<br>    description, paragraphs 6 and 15, and figure 8 | 4 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **01 June 2023** | **05 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/083705** |

### C.    DOCUMENTS CONSIDERED TO BE RELEVANT

| Category* | Citation of document, with indication, where appropriate, of the relevant passages | Relevant to claim No. |
| --- | --- | --- |
| Y | CN 108456713 A (TIANJIN NOVOGENE BIOINFORMATICS TECHNOLOGY CO., LTD.) 28 August 2018 (2018-08-28)<br>    description, paragraphs 21-23 | 4 |
| A | CN 106414770 A (IGENOMX INTERNAT GENOMICS CORP.) 15 February 2017 (2017-02-15)<br>    entire document | 1-18 |
| A | CN 109554447 A (WUHAN BIO-RAID BIOTECHNOLOGY CO., LTD.) 02 April 2019 (2019-04-02)<br>    entire document | 1-18 |
| A | CN 110396516 B (WUHAN SEQEALTH TECHNOLOGY CO., LTD.) 22 October 2021 (2021-10-22)<br>    entire document | 1-18 |
| A | CN 113463202 A (SEQUMED BIOTECH INC.; SOUTHERN MEDICAL UNIVERSITY) 01 October 2021 (2021-10-01)<br>    entire document | 1-18 |

Form PCT/ISA/210 (second sheet) (July 2022)

**INTERNATIONAL SEARCH REPORT**

| International application No. |
| --- |
| **PCT/CN2023/083705** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

| INTERNATIONAL SEARCH REPORT | | International application No. |
|---|---|---|
| Information on patent family members | | **PCT/CN2023/083705** |

| Patent document cited in search report | | | Publication date (day/month/year) | Patent family member(s) | | | Publication date (day/month/year) |
|---|---|---|---|---|---|---|---|
| CN | 113046835 | A | 29 June 2021 | None | | | |
| CN | 112553337 | A | 26 March 2021 | None | | | |
| CN | 101230490 | B | 30 January 2013 | None | | | |
| CN | 108456713 | A | 28 August 2018 | None | | | |
| CN | 106414770 | A | 15 February 2017 | JP | 2017512491 | A | 25 May 2017 |
| | | | | JP | 6996844 | B2 | 03 February 2022 |
| | | | | DK | 3110975 | T3 | 11 October 2021 |
| | | | | PL | 3110975 | T3 | 13 December 2021 |
| | | | | AU | 2021240134 | A1 | 28 October 2021 |
| | | | | WO | 2015131110 | A1 | 03 September 2015 |
| | | | | EP | 3110975 | A1 | 04 January 2017 |
| | | | | EP | 3110975 | A4 | 06 December 2017 |
| | | | | EP | 3110975 | B1 | 07 July 2021 |
| | | | | AU | 2015222802 | A1 | 13 October 2016 |
| | | | | AU | 2015222802 | B2 | 01 July 2021 |
| | | | | JP | 2020156491 | A | 01 October 2020 |
| | | | | EP | 3943615 | A1 | 26 January 2022 |
| | | | | US | 2016376663 | A1 | 29 December 2016 |
| | | | | ES | 2894048 | T3 | 11 February 2022 |
| | | | | CA | 2940669 | A1 | 03 September 2015 |
| CN | 109554447 | A | 02 April 2019 | None | | | |
| CN | 110396516 | B | 22 October 2021 | None | | | |
| CN | 113463202 | A | 01 October 2021 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210303736
- WO 2014130635 A
- WO 2013176916 A
- WO 2013176915 A
- WO 2014039523 A
- WO 2014184741 A
- WO 2014191128 A
- WO 2014184744 A
- WO 2014184143 A

**Non-patent literature cited in the description**

- **HACEIN-BEY-ABINA S et al.** *science*, 2003, vol. 302 (5644), 415-419
- **SIX E et al.** Molecular Therapy. CELL PRESS, 2017, vol. 25, 347-348
- Bluebird bio reports second quarter financial results and provides operational update. *bluebirdbio*, 09 August 2021
- **O'LEARY M C et al.** *Clinical Cancer Research*, 2019, vol. 25 (4), 1142-1146